Europäisches Patentamt

European Patent Office

Office européen des brevets

(11) Veröffentlichungsnummer: **0 342 443**
**A1**

## (12) EUROPÄISCHE PATENTANMELDUNG

(21) Anmeldenummer: 89108087.1

(22) Anmeldetag: 05.05.89

(51) Int. Cl.⁴: **A61M 16/00** , **A61M 16/01** , **G05B 17/00**

(30) Priorität: 19.05.88 DE 3817053

(43) Veröffentlichungstag der Anmeldung:
23.11.89 Patentblatt 89/47

(84) Benannte Vertragsstaaten:
**DE FR GB SE**

(71) Anmelder: **Drägerwerk Aktiengesellschaft**
**Moislinger Allee 53-55**
**D-2400 Lübeck 1(DE)**

(72) Erfinder: **Bayerlein, Jörg, Dr. Dipl.-Ing.**
**Schulweg 4d**
**D-2406 Stockelsdorf(DE)**

(54) **Verfahren zur Steuerung eines Beatmungsgerätes und Vorrichtung hierzu.**

(57) Ein Verfahren zur Steuerung eines Beatmungsgerätes, bei dem eine nach Patientendaten gewählte Einstellung nach einer Einwirkungszeit zur Optimierung verändert werden soll, ist dadurch gekennzeichnet, daß eine die Kennwerte des Beatmungsgerätes (2) nachbildende erste Simuliereinheit (6) (Modell des Beatmungsgerätes) und eine aus den Patientendaten die Patientenparameter nachbildende zweite Simuliereinheit (7) (Patientenmodell) mit dem Beatmungsgerät (2) derart verbunden sind, daß die vorgesehene neue Einstellung bei unveränderter Einstellung des Beatmungsgerätes (2) zuerst an der ersten Simuliereinheit (6) durchgeführt wird, daß die resultierenden Ausgabewerte der ersten Simuliereinheit mit den Patientendaten der zweiten Simuliereinheit (7) derart verknüpft werden, daß die Auswirkung der geänderten Einstellung auf den Patienten als simulierte neue Patientendaten ableitbar wird, und daß eine Umschaltung des Beatmungsgerätes auf die neue Einstellung durch Steuerbefehl (4) erfolgen kann.

EP 0 342 443 A1

## Verfahren zur Steuerung eines Beatmungsgerätes und Vorrichtung hierzu

Die Erfindung betrifft ein Verfahren zur Steuerung eines Beatmungsgerätes, bei dem eine nach Patientendaten gewählte Einstellung nach einer Einwirkungszeit zur Optimierung verändert werden soll.

Bei der Einstellung von Beatmungsgeräten,insbesondere in der Intensivpflege von Patienten werden zunächst bestimmte Einstellwerte nach dem vorliegenden Zustandsbild eingestellt, und es wird beobachtet, ob bei einer Beatmung mit diesen Werten eine verbesserte physiologische Gesamtsituation bzw. eine Stabilisierung des Atmungssystems erreicht werden kann. Da die erste Einstellung in vielen Fällen nachträglich verändert werden muß, und da eine solche Veränderung oft zu nicht vorhersehbaren und teilweise gefährlichen Reaktionen des Patienten führen kann, besteht der Wunsch, die Auswirkungen einer Neueinstellung wenigstens abschätzungsweise zu kennen, ehe eine entsprechende Veränderung in der Grundeinstellung des Beatmungsgerätes durchgeführt wird.

Zum bekannten Stande der Technik gehört ein Narkosesimulator, wie er in der Literaturstelle "Abott-Narkosesimulator" von Helmut Schwilden (deutsche Abott GmbH, Dezember 1986) beschrieben ist. Dort wird mit Hilfe eines Rechners die mögliche Verdampfereinstellung des Narkosegerätes mit den Parametern des Beatmungssystems und gewissen physiologischen Größen des Patienten verknüpft. Nach Einstellung der für die Narkose wesentlichen Daten des Patienten und des Narkosegerätes wird das Inhalationsanästhetikum ausgewählt und in seiner Dosierung festgelegt. Eine solche Simulation ermöglicht eine patientenangepaßte Auswahl des Narkosemittels und die Festlegung der hierfür erforderlichen Dosierung sowie der zusätzlichen Beatmungsgrößen. Sie erlaubt ferner die Anzeige eines zeitlichen Profils für den zu erwartenden Narkoseablauf. Diese Simulation wird jedoch nicht während des Narkoseablaufs durchgeführt, sondern sie dient der Ermittlung der im späteren Narkoseverfahren zu wählenden Einstellgrößen und der zweckmäßigen Auswahl des Narkosemittels.

Die Erfindung geht von der Aufgabenstellung aus, ein Verfahren zur Steuerung eines Beatmungsgerätes so auszubilden, daß während der Durchführung der Beatmung vor einer Änderung der getroffenen Einstellung eine versuchsweise Änderung fiktiv durchgeführt werden kann, welche eine Abschätzung hinsichtlich der als Folge der geänderten Einstellung zu erwartenden geänderten Patientendaten ermöglicht.

Die Lösung dieser Aufgabenstellung besteht darin, daß eine die Kennwerte des Beatmungsgerätes nachbildende erste Simuliereinheit (Modell des Beatmungsgerätes) und eine aus den Patientendaten die Patientenparameter nachbildende zweite Simuliereinheit (Patientenmodell) mit dem Beatmungsgerät derart verbunden sind, daß die vorgesehene Neueinstellung bei unveränderter Einstellung des Beatmungsgerätes zunächst an der ersten Simuliereinheit durchgeführt wird, daß die resultierenden Ausgabewerte der ersten Simuliereinheit mit den Patientendaten der zweiten Simuliereinheit derart verknüpft werden, daß die Auswirkung der geänderten Einstellung auf den Patienten als simulierte neue Patientendaten ableitbar wird, und daß eine Umschaltung des Beatmungsgerätes auf die neue Einstellung durch Steuerbefehl erfolgen kann.

Ein solches Verfahren, bei dem Beatmungsgerät und Patient in einer entsprechenden Mikroprozessoreinheit als Modell nachgebildet werden, ermöglicht eine Abschätzung der als Folge der Neueinstellung resultierenden neuen Patientendaten, ohne daß damit irgendeine Beeinträchtigung des mit der ursprünglichen Einstellung weiterbeatmeten Patienten erfolgen kann. Erweist sich die Neueinstellung als zweckmäßig, d.h. werden gegenüber der ursprünglichen Einstellung optimalere Patientendaten als Erwartungswerte erreicht, so kann durch Steuerbefehl,beispielsweise über einen handbetätigbaren Schalter,die Umschaltung des Beatmungsgerätes auf die neue Einstellung erfolgen. Es ist ferner möglich, nach der Simulation der Neueinstellung auch wie bisher das Beatmungsgerät von Hand auf die neuen Beatmungsdaten einzustellen.

Eine wichtige Verbesserung kann gegebenenfalls dadurch erreicht werden, daß der zweiten Simuliereinheit ( Patientenmodell) fortgesetzt die Patientendaten zugeführt werden, und daß aus diesen Patientendaten die Parameter für das Patientenmodell und damit die Simulation verbessert wird. Dadurch läßt sich das Reaktionsverhalten des Patienten in gewisser Weise in sein zugeordnetes Patientenmodell einbringen und durch fortgesetzte Verbesserung wird auch die Abschätzung von Auswirkungen bei einer simulierten Neueinstellung des Beatmungsgerätes zuverlässiger.

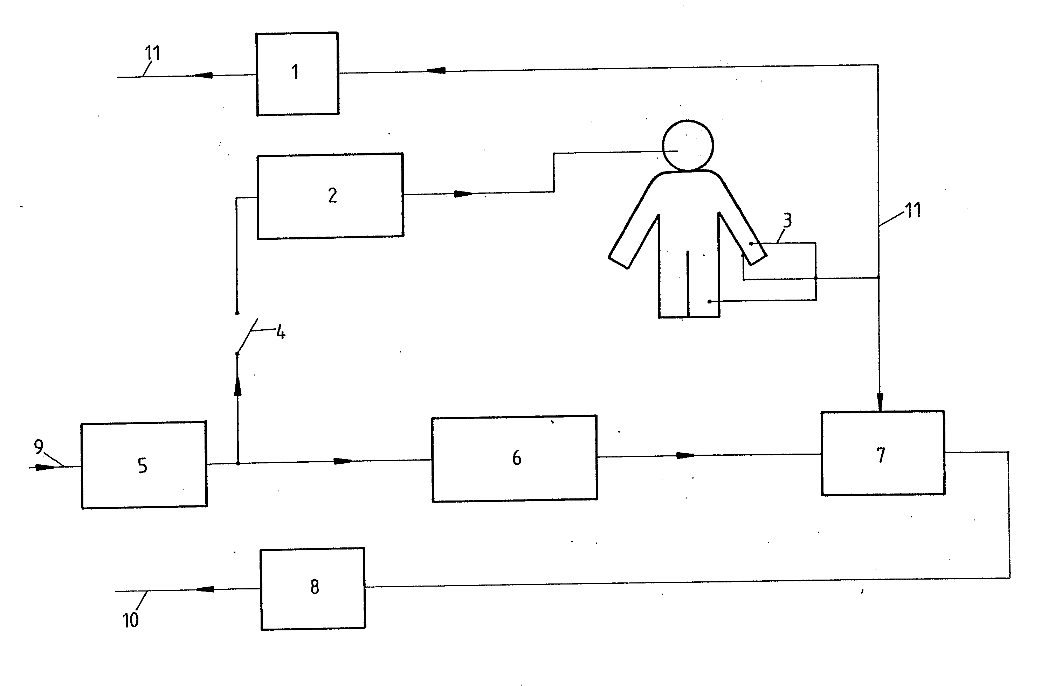

In der Zeichnung ist eine schematische Schaltungsanordnung zur Durchführung des erfindungsgemäßen Verfahrens dargestellt, aus der sich weitere Erfindungsmerkmale ergeben.

Das Verfahren geht von einem als Stand der Technik bekannten Beatmungsgerät, wie es beispielsweise in der DE-PS 27 46 924 beschrieben ist, aus. Dieses Beatmungsgerät 2 beatmet einen Patienten und die über Patientensensoren 3 abgenommenen Meßwerte werden als aktuelle Patiententdaten 11 mit einer ersten Anzeigeeinheit 1 angezeigt. Die vom Patienten mit bekannten Meßgeräten abgenommenen Meßwerte sind beispielsweise Atemdruckwerte, Atemflow, gegebenenfalls aber auch Werte der Blutgaskonzentrationen. Diese bekannte Grundeinheit wird nun derart verändert, daß eine vom Beatmungsgerät 2 getrennte Einstelleinheit 5 vorhanden ist, die es erlaubt, das Beatmungsgerät hinsichtlich seiner Einstellwerte 9, wie Atemvolumen, Frequenz und dergleichen zu verstellen bzw. einzustellen. Die Einstelleinheit 5 ist vom Beatmungsgerät durch einen Steuerschalter 4 getrennt, welcher beim Schließen den Steuerbefehl zur Übernahme der Einstellwerte aus derEinstelleinheit 5 in das Beatmungsgerät 2 erteilt.

Bei geöffnetem Schalter 4 gelangen die gewünschten oder vom Benutzer ausgewählten Einstellwerte 9 zunächst nicht zum Beatmungsgerät 2, sondern zu einer die Kennwerte des Beatmungsgerätes nachbildenden ersten Simuliereinheit 6. Diese Simuliereinheit 6 erzeugt ein fiktives Ausgangssignal, das nunmehr auf eine den Patienten nachbildende zweite Simuliereinheit 7 einwirkt. Diese zweite Simuliereinheit 7 kann z.B. ein Lungenmodell mit den Parametern Resistance und Compliance sein; die fiktiven Ausgangswerte sind in diesem Falle Druck und Flow. Das Patientenmodell liefert dann als Ausgangsgröße simulierte Meßwerte 10, die über eine zweite Anzeigeeinheit 8 als simulierte Folgewerte dem Anwender angezeigt werden.

Der Anwender kann nun bei geöffnetem Steuerschalter 4 die Einstellung an der Einstelleinheit 5 beliebig verändern, ohne auf die aktuelle Beatmung des Patienten einzuwirken. Er kann sich einen gewünschten fiktiven Patientenwert einstellen, der seinem Therapieziel nahekommt, und kann praktisch üben und das Verhalten des simulierten Patienten, wie des simulierten Beatmungsgerätes studieren. Der Vorteil liegt z.B. darin, daß die Verweilzeit des Patienten, z.B. in der Intensivpflege deutlich reduziert werden kann, so daß Fehleinstellungen nicht erst dann erkannt werden, wenn sie sich bereits auf den Patienten schädlich auswirken, sondern bereits vorher ohne Reaktion des Patienten als simuliertes Fehlverhalten.

Die Zuverlässigkeit der Simulation hängt selbstverständlich von der Güte der Modelle, d.h. des Modells des Beatmungsgerätes und des Patientenmodells ab. Da die erste Simuliereinheit 6 ein technisches Gerät simuliert, ist diese Simulation einfacher zu realisieren. Es ist allerdings zu berücksichtigen, daß die zweite Simuliereinheit 7, d.h. das Patientenmodell auch Rückwirkungen auf die erste Simuliereinheit 6 (Modell des Beatmungsgerätes) haben kann. Zum Beispiel wird bei der Applikation eines bestimmten Tidalvolumens je nach den Lungencharakteristika eine Druckbegrenzung einsetzen, die das Zuführen des gewünschten Tidalvolumens verhindert.

In der Regel lassen sich solche Eigenschaften aber aus den technischen Beschreibungen der Beatmungsgeräte entnehmen, so daß eine aktive Wirkverbindung zwischen dem Beatmungsgerät 2 und der ersten Simuliereinheit 6 nicht nötig ist.

Anders dagegen verhält sich die zweite Simuliereinheit 7 für das Patientenverhalten. Hier ist es möglich, aus den Patientendaten die als Meßwerte 11 abgenommen werden, das tatsächliche Patientenverhalten zu beschreiben. Durch geeignete Identifikationsverfahren lassen sich dann Parameter für das Patientenmodell gewinnen bzw. in ihrer Größe und in ihrem Trend abschätzen. So ist es z.B. möglich, anhand des Verlaufes von Druck und Flow des aufgenommenen Atemgases die Lungencharakteristika Resistance und Compliance zu bestimmen, so daß ein lineares physikalisches Lungenmodell für die zweite Simuliereinheit 7 verwendet werden kann.

Ist der Benutzer mit den von den Simuliereinheiten erzeugten fiktiven Folgewerten einverstanden, dann kann er durch Schließen des Steuerschalters 4 die neuen Einstellwerte an das Beatmungsgerät 2 weiterleiten und die tatsächliche Auswirkung der Einstellung auf den Patienten anhand der ersten Anzeigeeinheit 1 beobachten. Sieht er beide Anzeigen 1 und 8 gleichzeitig oder sogar in einer geeigneten Darstellung auf dem gleichen Bildschirm übereinander, so kann er sofort Rückschlüsse auf die Güte der Simulation ziehen.

Im Gegensatz zu bekannten Simulatoren, wie z.B. Flugsimulatoren, kann der Anwender mit dem hier beschriebenen Verfahren unmittelbar bei in der Routine laufendem Beatmungsvorgang mögliche Auswirkungen von Einstellungsänderungen prüfen, ohne diese zunächst mit Auswirkung auf den Patienten vorgenommen zu haben.

## Ansprüche

1. Verfahren zur Steuerung eines Beatmungsgerätes, bei dem eine nach Patientendaten gewählte Einstellung nach einer Einwirkungszeit zur Optimierung verändert werden soll, **dadurch gekennzeichnet,** daß eine die Kennwerte des Beatmungsgerätes (2) nachbildende erste Simuliereinheit (6) (Modell des Beatmungsgerätes) und eine aus den Patientendaten die Patientenparameter nachbildende zweite Simuliereinheit (7) (Patientenmodell) mit dem Beatmungsgerät (2) derart verbunden sind, daß die vorgesehene neue Einstellung bei unveränderter Einstellung des Beatmungsgerätes (2) zuerst an der ersten Simuliereinheit (6) durchgeführt wird, daß die resultierenden Ausgabewerte der ersten Simuliereinheit (6) mit den Patientendaten der zweiten Simuliereinheit (7) derart verknüpft werden, daß die Auswirkung der geänderten Einstellung auf den Patienten als simulierte neue Patientendaten ableitbar wird, und daß eine Umschaltung des Beatmungsgerätes (2) auf die neue Einstellung durch Steuerbefehl (4) erfolgen kann.

2. Verfahren nach Anspruch 1, **dadurch gekennzeichnet**, daß der zweiten Simuliereinheit (7) fortgesetzt die Patienten daten zugeführt werden, und daß aus diesen Patientendaten die Parameter für das Patientenmodell und damit die Simulation verbessert wird.

3. Schaltungsanordnung zur Durchführung des Verfahrens nach Anspruch 1, **dadurch gekennzeichnet**. daß die Ausgänge der Patientensensoren (3) mit einer ersten Anzeigeeinheit (1) und mit der zweiten Simuliereinheit (7) (Patientenmodell) verbunden sind, daß eine Einstelleinheit (5) vorgesehen ist, welche über einen Steuerschalter (4) mit dem Beatmungsgerät (2) und direkt mit der ersten Simuliereinheit (6) (Modell des Beatmungsgerätes) verbunden ist, daß der Ausgang der ersten Simuliereinheit (6) mit dem Eingang der zweiten Simuliereinheit (7) verbunden ist, und daß der Ausgang der zweiten Simuliereinheit (7) mit einer zweiten Anzeigeeinheit (8) verbunden ist.

Europäisches Patentamt

**EUROPÄISCHER RECHERCHENBERICHT**

Nummer der Anmeldung

EP 89 10 8087

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | GB-A-2 093 218 (MANUFACTURAS MEDICAS)<br>* Seite 1, Zeilen 17-64; Seite 2, Zeilen 41 -51; Anspruch 6 *<br>--- | 1-3 | A 61 M 16/00<br>A 61 M 16/01<br>G 05 B 17/00 |
| X | DD-A- 251 706 (INGENIEUR HOCHSCHULE DRESDEN)<br>* Insgesamt *<br>--- | 1-3 | |
| A | TRANS. OF INST. OF MEAS. & CONTROL., Band 2, Nr. 1, Januar/März 1980, Seiten 38-45, Dorking, GB; R.T. CHILCOAT: "A review of the control of depth of anaesthesia"<br>* Seite 40, Spalte 2, Zeile 28 - Seite 41, Spalte 1, Zeile 13; Figur 6 *<br>----- | 1 | |

**RECHERCHIERTE SACHGEBIETE (Int. Cl.4)**

A 61 M

Der vorliegende Recherchenbericht wurde für alle Patentansprüche erstellt

| Recherchenort | Abschlußdatum der Recherche | Prüfer |
|---|---|---|
| DEN HAAG | 02-08-1989 | SCHOENLEBEN J.E.F. |